# EUROPEAN PATENT SPECIFICATION

(11) **EP 2 054 070 B1**
(45) Date of publication and mention of the grant of the patent: **21.08.2013**
(21) Application number: 08751416.2
(22) Date of filing: 15.02.2008
(51) Int. Cl.: A61K 36/37, A61P 3/10

(54) **A NOVEL HERBAL DRUG AND A PROCESS FOR PREPARATION THEREOF FOR THE PREVENTION AND MANAGEMENT OF ENDOTHELLAL DYSFUNCTION AMONG TYPE-II DIABETES MELLITUS CASES**
NEUARTIGER KRÄUTERWIRKSTOFF UND VERFAHREN ZU SEINER HERSTELLUNG ZUR VORBEUGUNG UND BEHANDLUNG VON ENDOTHEL-DYSFUNKTIONEN IN TYP-II-DIABETES-MELLITUS-FÄLLEN
NOUVEAU MÉDICAMENT PHYTOTHÉRAPEUTIQUE ET SON PROCÉDÉ DE PRÉPARATION POUR LA PRÉVENTION ET LA GESTION D'UN DYSFONCTIONNEMENT ENDOTHÉLIAL PARMI DES CAS DE DIABÈTE DE TYPE II

(30) Priority: 15.02.2007 IN CH03142007
(43) Date of publication of application: 06.05.2009
(73) Proprietor: Swaminathan. S., Thanjavur 613 402 (IN)
(72) Inventor: DUBEY, G., P., Banaras Hindu University, Varanasi (IN); RAJAMANICKAM, G., Victor, Thanjavur (IN); SINGH, R., G., Hindu University (IN); AGRAWAL, Aruna, Banaras Hindu University, Varanasi (IN); VYAS, Neera, New Delhi (IN)
(74) Representative: Green, Mark Charles
(86) International application number: PCT/IN2008/000089
(87) International publication number: WO 2008/099423

(56) References cited:
- EP-A1- 1 645 557
- US-A1- 2002 041 904
- T HSUNWEIHUANG ET AL: "Salacia oblonga root improves postprandial hyperlipidemia and hepatic steatosis in Zucker diabetic fatty rats: Activation of PPAR-[alpha]", TOXICOLOGY AND APPLIED PHARMACOLOGY, vol. 210, no. 3, 1 February 2006 (2006-02-01), pages 225-235, XP55029828, ISSN: 0041-008X, DOI: 10.1016/j.taap.2005.05.003
- KRISHNAKUMAR K ET AL: "Hypolipidaemic effect of Salacia oblonga Wall. root bark in streptozotocin diabetic rats", MEDICAL SCIENCE RESEARCH, ELSEVIER APPLIED SCIENCE, BARKING, GB, vol. 28, no. 1, 1 January 2000 (2000-01-01), pages 65-67, XP009145763, ISSN: 0269-8951
- HUANG T H W ET AL: "Salacia oblonga root improves cardiac lipid metabolism in Zucker diabetic fatty rats: Modulation of cardiac PPAR-alpha-mediated transcription of fatty acid metabolic genes", TOXICOLOGY AND APPLIED PHARMACOLOGY, ACADEMIC PRESS, US, vol. 210, no. 1-2, 1 January 2006 (2006-01-01), pages 78-85, XP024895967, ISSN: 0041-008X, DOI: 10.1016/J.TAAP.2005.07.020 [retrieved on 2006-01-01]

## Description

### FIELD OF INVENTION

The present invention relates to the novel plant based preparation and to the process thereof for the prevention and management of endothelial dysfunction caused due to hyperglycemia among type-II diabetes mellitus cases. The preparation of present invention may be advantageous if used for the management of altered pro-inflammatory bio-markers, high endotheline, hyperleptinemia, hypo-adiponectinemia, atherogenic injury including prevention from oxidative injury among type-II diabetes mellitus cases.

### BACKGROUND OF INVENTION

The present study has been planned to investigate the role of organic extract of leaves and root of Salacia oblonga in the prevention and management of endothelial dysfunction caused due to hyperglycemia in type II diabetes mellitus cases. Type II diabetes is the predominant form of diabetes worldwide accounting for 90% of case globally. In type II diabetes, two impairments are found to increase blood glucose levels; impaired insulin action and impaired pancreatic insulin secretion. Diabetes is associated with factors which directly contribute to cardiovascular disorders including insulin resistance, dyslipidemia, hyperinsulinemia, hypertension, excessive oxidations, endothelial dysfunction and inflammation.

The most common and life threatening disorder among type-II diabetic subjects is coronary Heart disease. The diabetic condition Contributes for initiation and progression of micro and macro vascular complications. Hyperhomocysteinemia is recognized as independent risk factor for CVD and type II diabetes. Elevated plasma homocysteine level has been related to diabetes complications and prospective studies have suggested a stronger relationship between homocysteine and mortality in persons with diabetes than without diabetes.

High concentration of homocysteine may exert an atherothrombotic effect through increasing oxidative stress, which may induce endothelial dysfunction and also affect the properties of extra-cellular matrix and increase smooth muscle cell proliferation. Type-II diabetes mellitus is associated with a decrease in the bioavailability of endothelium derived nitric oxide and increased inflammation, which may be responsible for increase in atherosclerosis in diabetes. Diabetes is associated with more release of various pro-inflammatory adipo-kines that contribute to vascular inflammation and atherogenesis. Thus modulation of endothelial function and reducing the inflammation is important in diabetes to prevent atherosclerotic complications.

Over the last decade, it has been recognized that adipose tissue is an important endocrine organ that produces a number of hormones -Leptin, tumor necrosis factor-α, Plasminogen activator inhibitor-I, resistin and adiponectin - with essential roles in the regulation of body weight, insulin sensitivity and glucose metabolism as well as other physiologic functions.

Leptin is a hormone, with interleukin-6 homology it acts on a specific receptor located in the hypothalamus to decrease appetite and increase energy expenditure and thus it has been hypothesized as "adiposity signal" for the long term regulation of body weight by the brain. Leptin has been found to be positively associated with insulin resistance, diabetes risk, triglyceride levels, impaired fibrinolysis, c-reactive protein and blood pressure and inversely associated with high-density lipoprotein cholesterol level. Data from several studies suggests a strong positive correlation between leptin and insulin concentration, showing that insulin-resistant may have higher leptin concentrations than those who are insulin-sensitive.

Adiponectine, is a protein secreted by adipose cells may regulate insulin sensitivity with energy metabolism. Metabolic syndrome-x, diabetes, atherosclerosis, hypertension and coronary artery disease are associated with decreased plasma levels of adiponectin, insulin resistance and endothelial dysfunction.

Tumor necrosis factor-α decreases tyrosine kinase activity of the insulin receptor and is overproduced in adipose tissues in insulin-resistant rodents and humans suggesting that it is a possible mediator of insulin resistance in obesity and diabetes.

Recently, some of the biomarkers are emerging found useful in the diagnosis and treatment of the pathophysiology of cardiac disease such as cardiac troponin (cTn). The cTn elevations are common in patients with a large number of acute and chronic cardio vascular diseases. The elderly cases having chest pain at rest, transient electrocardiogram changes, increasing symptoms or signs of ischemia, evidence of hemodynamic instability and arrhythmia have shown high incidence of elevated cTn.

More recently, 3CD40 ligand which is a signaling protein, reflects both inflammatory and platelet interactions with the plaque. An elevated 3CD40 ligand is found in patients with acute coronary syndrome

A number of anti-diabetic, anti-hypertensive, vasodilatory, lipid lowering agents are being used but the applications of such synthetic chemical agents are limited as their prolonged use are claimed to produce several side effects on biological system. Scientific evaluation of some of the Ayurvedic drugs have shown better efficacy over side effects of conventional therapy. Generally, it is observed that due to unsatisfactory response of the drugs and troublesome side effects the currently available drugs are not able to reduce the mortality and morbidity rate due to CHD particularly among diabetes. In preliminary observations some of the plant based drugs have shown blood glucose lowering, lipid lowering, anti-hypertensive, anti-obesity and anti-inflammatory properties without any risk profile. Salacia oblonga is an Ayurvedic plant which has shown beneficial role in the prevention and management of various bio-markers associated with hyperglycemia responsible for endothelial dysfunction and ultimately manifesting into an adverse cardiac event. Reduction in pro-inflammatory biomarkers like c-reactive protein, interleukin-6, TNF-α and abnormal lipid concentrations indicated the improvement in endothelial dysfunction and also improvement in atherothrombotic changes in type II diabetes mellitus cases following organic extract of Salacia Oblonga in effective doses.

### OBJECTS OF THE INVENTION

The main object of the present invention is to propose a novel herbal drug for the prevention and management of endothelial dysfunction among type-II diabetes mellitus cases.

The another object of the present invention is to propose a new herbal preparation for the management of atherogenic injury caused due to hyperglycemia among type-II diabetes mellitus.

Still another object of the present invention is to propose an herbal drug having potential role in the prevention and management of pro-inflammatory markers like interleukin-₆, c-reactive protein, TNF-α responsible for endothelial dysfunction.

Another object is to prevent the formation of atherothrombotic plaques responsible for cardiac and cerebral stroke.

Yet another object of the present invention is to propose an herbal formulation beneficial in the management of hyperhomocysteinemia associated with endothelial dysfunction among type-II diabetes mellitus.

Still another object of the present invention is to propose a novel herbal drug beneficial in reducing the leptin level among type-II diabetes mellitus.

Another object of the present invention is to propose an herbal formulation beneficial in the enhancing the adiponectin level among type-II diabetes mellitus cases.

Another object is to propose an herbal drug effective in reducing the endotheline among type-II diabetes mellitus cases.

Further, the object of the present invention is to propose an herbal formulation effective in the regulation of plasminogen activator inhibitor among type-II diabetes mellitus cases.

The object of the present invention is to propose an herbal formulation beneficial in the prevention and management of dyslipidemia particularly apolipo (B) among type-II diabetes mellitus cases.

The main object of the invention is to propose an herbal formulation effective in the management of risk factors responsible for cerebral and cardiac stroke, to improve the renal insufficiency, and endothelial dysfunction so that patients of type-II diabetes mellitus may be prevented form adverse cardiac event.

### STATEMENT OF INVENTION

According to this invention there is provided a novel herbal drug for the prevention and management of endothellal dysfunction among type-II diabetes mellitus cases comprising of 400-750 mg/day of extract of root and 200-400 mg/day of extract of leaves of salacia oblonga (sapttarangi).

Further according to this invention there is provided a process for preparation of a novel herbal drug for the prevention and management of endothelial dysfunction among type-II diabetes mellitus cases such as herein described.

### DETAILED DESCRIPTION OF THE INVENTION

The hydro-alcoholic extract of root and leaves of the plant Salacia oblonga was prepared by using for example 30:70 solvent i.e. 30% water and70% methanol. The water utilized for extraction is decontaminated for any type of bacterial or abnormal growth by using reverse osmosis plant. After extraction, the active molecule is identified and separated by HPLC, HPTLC and NMR procedure.

The biological activity is studied on the basis of mode of action of the test drug through its action of biological markers responsible for endothelial dysfunction due to hyperglycemia and insulin resistance. The molecular characterization is done by using NMR and bio-molecule reaction following the interaction between the chemical and biological markers responsible for endothelial dysfunction among type-II diabetes mellitus.

The pre-clinical safety and efficacy studies of both extract (root and leave) of Salacia oblonga is carried out. Both extracts are mixed in effective doses and further toxicological acute, sub-acute and chronic studies are conducted and safety profile is determined before utilizing the test drug for human trials. The mode of action of test drug is carried out in animal models.

The anti-inflammatory, anti-atherogenic and anti-oxidant effect of test drug is validated. The action of the test drug is also studied on homocysteine, leptin, adiponectin, endotheline and plasminogen activator inhibitor.

The test drug has shown beneficial role in the prevention and management of endothelial dysfunction through its direct effect on biological markers as well as through reducing the insulin load and glycemic control among diabetes mellitus cases.

According to this invention there is provided herbal formulation for the prevention and management of endothelial dysfunction associated with diabetes mellitus and responsible for adverse cardiac event. This invention is more concentrated on the vascular complications associated with diabetic patients with the object to prevent the coronary syndrome. The present herbal formulation comprising of extract of root and leaves of Salacia oblonga as given bellow -

| **Name of the plant** | **Dose range** |
|---|---|
| Salacia oblonga (Sapttarangi) Root | 400-750 mg/day |
| Salacia oblonga (Sapttarangi) Leaves | 200-400 mg/day |

The formulation may also comprise of a known additives such as minerals, vitamin, salts, fillers (for capsulation or to prepare syrup) and binders, if required to be present in trace amount.

| **Name of the plant** | **Part used** | **Dose range** |
|---|---|---|
| Salacia oblonga (Sapttarangi) | Root | 400-750 mg/day |
| Salacia oblonga (Sapttarangi) | Leaves | 200-400 mg/day |

Thus any known additives or supplement is added to prepare the final formulation if required and present in trace amount. Reference is made here in capsule form (500 mg each capsule). However, it would be apparent that the preparation may also be in the form of tablet.

**Preferably the preparation comprises:**

| **Name of the plant:** | **Doses** |
|---|---|
| Salacia oblonga (Sapttarangi) Root | 625 mg/day |
| Salacia oblonga (Sapttarangi) Leaves | 325 mg/day |

The above doses are given in combined form in two divided doses/day (425 mg morning and 425 mg evening)

### About the plant:

Salacia oblonga belongs to Celastraceae family. It is a large family of woody shrubs and trees with a Gondwanan distribution. The molecular modeling of Salacia oblonga is carried out with the view to know the biological property of the plant.

In Ayurvedic system of Medicine Salacia oblonga has been used for the treatment of diabetes. Salacinol and kotalanol are the constituents derived from root of Salacia oblonga, binds to intestinal enzyme called alpha-glucosidase which is responsible for the breakdown of polysaccharide in to monosaccharide in the body. Thus, it is a potent alpha-glucosidase inhibitor. In several recent studies the leaves of Salacia oblonga has shown anti-inflammatory properties The leaves contains several biologically active constituents i.e. azimine, azecarpin, carpine, friedelin, lupeol, glutinol and β-sitosterol.

Investigation has been carried out regarding the therapeutic use of Salacia oblonga by proving the alpha glucosidase inhibiting property of the plant. The anti-inflammatory role of this plant is studied by, in animal model by carrageenan-induced rat paw oedema and cotton pellet granuloma method.

Further, the anti-oxidant effects are also evaluated by **Li Y et al (2004).** Findings have been made regarding the effect of Salacia oblonga in the prevention of cardiac fibrosis and postprandial hyperglycemia in obese zucker rats. Further, evaluation has been done regarding the effect of different doses of Salacia oblonga extract on postprandial glycemia, insulinemia and breath hydrogen response in healthy adults. Recently, studies were made on the root extract of Salacia oblonga in animal models and concluded no observable adverse effect even at the dose of 2500 mg/kg/day by oral gavage administrations.

From the above, it is evident that no work has been done on the role of Salacia oblonga in the prevention and management of endothelial dysfunction responsible for renal-cardiac and cerebral damage following hyperglycemia/insulin resistance. In our study the drug has shown specific action on insulin receptors and leptin receptor and improves the level of adiponectin which prevents the atherosclerosis and protects the endothelial dysfunction. Root and leaves of Salacia oblonga have been utilized for therapeutic potentials.

### A novel research out come

### Decrease in Interleukin-₆ Following Test drug Treatment in Pre-diabetes cases

| Treatment Groups | Sex | No. of Cases | Interleukin 6 (pg/ml) | | | Comp. Initial vs After 6 months treatment | |
|---|---|---|---|---|---|---|---|
| | | | Initial | After 3 Months Treatment | After 6 months Treatment | T Value | P Value |
| Placebo Treated | M | 31 | 1.04 ±0.20 | 1.17 ±0.18 | 1.14 ± 0.21 | T=1.92 | P>0.05 |
| | F | 20 | 1.08 ± 0.24 | 1.09 ±0.18 | 0.98 ± 0.31 | T=1.14 | P>0.05 |
| Treated with Test Formulation | M | 32 | 1.22 ± 0.23 | 0.85 ± 0.34 | 0.71 ± 0.22 | T=9.10 | P<0.001 |
| | F | 23 | 1.32 ± 0.21 | 0.96 ±0.27 | 0.81 ± 0.22 | T=8.09 | P<0.001 |

| | | | | | | | |
|---|---|---|---|---|---|---|---|
| Normal range: <1 pg/ml | | | | | | | |

### Effect of Test drug on CRP Level in Pre-diabetes cases

| Treatment Groups | Sex | No. of Cases | CRP Level (mg/L) | | | Comp. Initial vs After 6 months treatment | |
|---|---|---|---|---|---|---|---|
| | | | Initial | After 3 Months Treatment | After 6 months Treatment | T Value | P Value |
| Placebo Treated | M | 31 | 1.57 ±0.40 | 1.61 ±0.25 | 1.68 ± 0.36 | T=1.14 | P>0.05 |
| | F | 20 | 1.41 ± 0.45 | 1.45 ± 0.34 | 1.42 ±0.39 | T=0.75 | P>0.05 |
| Treated with Test Formulation | M | 32 | 1.75 ± 0.42 | 1.38 ±0.39 | 1.12 ± 0.29 | T=7.00 | P<0.001 |
| | F | 23 | 1.93 ± 0.42 | 1.48 ±0.41 | 1.03 ± 0.32 | T=8.18 | P<0.001 |

| | | | | | | | |
|---|---|---|---|---|---|---|---|
| Normal range: 1-3 mg/L | | | | | | | |

### Decrease in Inflammatory Biomarker TNFα Following Test drug Treatment in Pre-diabetes cases

| Treatment Groups | Sex | No. of Cases | TNF-α (pg/mL) | | | Comp. Initial vs After 6 months treatment | |
|---|---|---|---|---|---|---|---|
| | | | Initial | After 3 Months Treatment | After 6 months Treatment | T Value | P Value |
| Placebo Treated | M | 31 | 162.41 ± 30.13 | 157.39 ± 38.44 | 159.75 ± 33.91 | T=0.32 | P>0.05 |
| | F | 20 | 171.20 ± 25.73 | 168.96 ± 31.04 | 173.75 ± 35.08 | T=0.26 | P>0.05 |
| Treated with Test Formulation | M | 32 | 168.97 ± 31.64 | 134.73 ± 25.13 | 121.91 ±18.99 | T=7.21 | P<0.001 |
| | F | 23 | 182.73 ± 38.11 | 148.96 ± 29.75 | 136.94 ± 23.84 | T=4.88 | P<0.001 |

| | | | | | | | |
|---|---|---|---|---|---|---|---|
| Normal range: 25-800 pg/ml | | | | | | | |

### Beneficial Role of the Test drug on Endotheline Level among Pre-diabetes cases

| Treatment Groups | Sex | No. of Cases | Endotheline (pg/mL) | | | Comp. Initial vs After 6 months treatment | |
|---|---|---|---|---|---|---|---|
| | | | Initial | After 3 Months Treatment | After 6 months Treatment | T Value | P Value |
| Placebo Treated | M | 31 | 413.91 ±63.54 | 420.84 ± 71.90 | 418.94 ±73.84 | T=0.28 | P>0.05 |
| | F | 20 | 338.75 ± 41.97 | 346.22 ±53.17 | 355.81 ± 58.27 | T=1.06 | P>0.05 |
| Treated with Test Formulation | M | 32 | 478.89 ± 64.34 | 409.64 ±52.14 | 373.91 ± 35.87 | T=8.06 | P<0.001 |
| | F | 23 | 371.93 ±51.34 | 338.87 . ± 48.90 | 283.92 ± 58.25 | T=5.43 | P<0.001 |

| | | | | | | | |
|---|---|---|---|---|---|---|---|
| Normal range: 0.32-1000 pg/ml | | | | | | | |

### Decrease in Apolipo B Following Test drug Treatment in Pre-diabetes cases

| Treatment Groups | Sex | No. of Cases | Apolipo B (mg/dL) | | | Comp. Initial vs After 6 months treatment | |
|---|---|---|---|---|---|---|---|
| | | | Initial | After 3 Months Treatment | After 6 months Treatment | T Value | P Value |
| Placebo Treated | M | 31 | 97.97 ± 12.21 | 98.39 ± 15.97 | 94.92 ± 13.82 | T=0.92 | P>0.05 |
| | F | 20 | 78.31 ± 16.75 | 73.14 ± 8.72 | 75.45 ± 14.87 | T=0.57 | P>0.05 |
| Treated with Test Formulation | M | 32 | 102.99 ± 32.42 | 92.90 ± 25.82 | 85.78 ±30.01 | T=2.20 | P<0.05 |
| | F | 23 | 96.72 ± 27.08 | 89.84 ± 24.88 | 80.72 ±17.91 | T=2.36 | P<0.05 |

| | | | | | | | |
|---|---|---|---|---|---|---|---|
| Normal range: 55-159 mg/dl | | | | | | | |

### Beneficial Role of the Test drug in Decreasing the Homocysteine Level among Pre-diabetes cases

| Treatment Groups | Sex | No. of Cases | Homocysteine (µmol/L) | | | Comp. Initial vs After 6 months treatment | |
|---|---|---|---|---|---|---|---|
| | | | Initial | After 3 Months Treatment | After 6 months Treatment | T Value | P Value |
| Placebo Treated | M | 31 | 13.79 ±4.11 | 14.24 ± 3.89 | 14.91 ±4.24 | T=1.05 | P>0.05 |
| | F | 20 | 14.82 ±3.31 | 13.64 ±5.91 | 15.02 ±3.32 | T=0.19 | P>0.05 |
| Treated with Test Formulation | M | 32 | 17.02 ± 4.92 | 15.71 ±4.06 | 12.94 ±3.75 | T=3.74 | P<0.001 |
| | F | 23 | 15.85 ±3.17 | 13.04 ±4.21 | 11.42 ± 3.81 | T=4.30 | P<0.001 |

| | | | | | | | |
|---|---|---|---|---|---|---|---|
| Normal range: 5-15 µmol/L | | | | | | | |

### Beneficial Role of the Test drug in decreasing the Plasminogen Activator inhibitor -1 Level among Pre-diabetes cases

| Treatment Groups | Sex | No. of Cases | Plasminogen activator inhibitor-1 (ng/ml) | | | Comp. Initial vs After 6 months treatment | |
|---|---|---|---|---|---|---|---|
| | | | Initial | After 3 Months Treatment | After 6 months Treatment | T Value | P Value |
| Placebo Treated | M | 24 | 30.71 ±4.03 | 29.93 ±3.19 | 31.04 ±4.11 | T=0.28 | P>0.05 |
| | F | 16 | 28.77 ±3.19 | 26.93 ±2.88 | 27.84 ±3.61 | T=0.77 | P>0.05 |
| Treated with Test Formulation | M | 29 | 29.84 ±3.20 | 26.73 ±2.85 | 24.91 ±4.01 | T=5.18 | P<0.001 |
| | F | 18 | 30.81 ±4.11 | 28.41 ±3.08 | 25.13 ±3.06 | T=4.73 | P<0.001 |

| | | | | | | | |
|---|---|---|---|---|---|---|---|
| Normal range: 15-25ng/ml | | | | | | | |

### Effect of Test drug on Tissue Plasminogen antigen Level in Pre-diabetes cases

| Treatment Groups | Sex | No. of Cases | Tissue Plasminogen antigen (ng/ml) | | | Comp. Initial vs After 6 months treatment | |
|---|---|---|---|---|---|---|---|
| | | | Initial | After 3 Months Treatment | After 6 months Treatment | T Value | P Value |
| Placebo Treated | M | 24 | 14.31 ±2.19 | 14.94 ±2.24 | 15.02 ±2.44 | T=1.07 | P>0.05 |
| | F | 16 | 15.01 ±3.14 | 14.87 ±2.82 | 14.97 ±2.90 | T=0.03 | P>0.05 |
| Treated with Test Formulation | M | 29 | 15.87 ±3.17 | 13.34 ±2.84 | 12.27 ±2.19 | T=5.07 | P<0.001 |
| | F | 18 | 14.77 ±3.12 | 12.08 ±2.16 | 10.64 ±2.45 | T=4.44 | P<0.001 |

| | | | | | | | |
|---|---|---|---|---|---|---|---|
| Normal range: 6-90 ng/ml | | | | | | | |

**Example-I:** When the hydro-alcoholic extract of root of Salacia oblonga in the dose of 30-60 mg/kg body wt. was orally administered to streptozotocin induced experimental rats, the drug exhibited alpha glucosidase inhibitory property and reduction in insulin load. The anti-oxidant and anti-inflammatory effects were also established in animal models.

**Example-II:** The test drug containing hydro-alcoholic extract of root (30-50 mg/kg/day) and leaves (25-45 mg/kg/day) was given for a period of one month to streptozotocin induced rats and the drug exerted improvement in inflammatory markers particularly CRP, IL-6 and TNFα. The drug has also shown anti-oxidant effects as it reduced the catalase and lipid peroxidase activity among streptozotocin induced oxidative injury. Glutathione level increased due to its anti-oxidant property thus indicating that test drug has potentiality to check the further development of diabetes.

**Example-III:** The test drug containing the hydro-alcoholic extract of Salacia oblonga, root in the dose range of (30-45 mg/kg/day) and leaves in the dose range of (20-30 mg/kg/day), to streptozotocin induced animals. An increase in nitric oxide, prostacycline and aenosine were noticed. The result indicated the vasodilatation property of the drug.

**Example-IV:** The hydro-alcoholic extract of Salacia oblonga, root (375-550 mg/day) and leaves (250-425 mg/day) was mixed and orally administered to cases of type-II diabetes mellitus which showed improvement in endothelial dysfunction as level of endotheline markedly reduced and brachial artery thickening also reduced.

**Example-V:** The hydro-alcoholic extract of test drug (containing extract of both root and leaves), root in the dose range of (450-600 mg/day) and leaves in the dose range of (325-450 mg/day), was given to type-II diabetes mellitus cases total cholesterol, LDL-c, Apolipo (B) and triglycerides levels reduced suggesting improvement in atherogenic injury due to dyslipidemia.

**Example-VI:** When the hydro alcoholic extract of test formulation in the dose range of 525-725mg/day (root) and 200-325 mg/day (leaves), was given to diabetic subjects reduction in inflammatory markers like IL-6, CRP and TNF-α was recorded during 3 to 6 months of study period. The reduction in the pro-inflammatory markers suggested the improvement in endothelial dysfunction under treatment of novel test drug.

**Example-VII:** The test formulation containing the hydro-alcoholic extract of root of Salacia oblonga in the dose of 425-600 mg/day along with extract of Salacia oblonga leaves in the dose of 275-425 mg/day was given to type-II diabetes mellitus cases exerted reduction in postprandial blood glucose level and glycosylated hemoglobin content. It is observed that test drug reduces the glycemic index by reducing insulin load among the diabetics.

**Example-VIII:** High level of leptin and low values of adiponectin was recorded in most of diabetic cases before therapy. The extract of Salacia oblonga root (525-725 mg/day) and leaves (225-350 mg/day) exerted better results in reducing the leptin and increasing the adiponectin levels in diabetes cases. A moderate decrease in BMI and improvement in insulin resistance was also recorded following test drug treatment.

**Example-IX:** In few diabetes cases elevated cTN and 3CD40 ligand were measured indicating grater risk of CHD. The test drug treatment in the range of 450-680 mg/day (root) and 325-450 mg/day (leaves) reduced the levels of above parameters indicating reduction in risk of CHD events among diabetes patients.

**Example-X:** The organic extract of root in the dose range of 525-775 mg/day and leaves in the dose range of 275-425 mg/day exerted most beneficial effects in the management of endothelial dysfunction in type-II diabetes mellitus by acting on various inflammatory markers including leptin and adiponectin level, endotheline, cTN, 3CD40 ligand risk markers of CHD associated with type-II diabetes mellitus. The test drug prevented the diabetics from cerebral and coronary stroke by preventing the formation of atherothrombotic plaques, thus, prevented from adverse cardiac event.

It is proposed that test formulation is effective in the prevention and management of endothelial dysfunction among diabetes mellitus cases.

It is to be noted that the present invention is susceptible to modifications, adaptations and changes by those skilled in the art. Such variant embodiments employing the concepts and features of this invention are intended to be within the scope of the present invention, which is further set forth under the following claims:-

## Claims

1. A composition for use in the prevention and management of endothelial dysfunction among type-II diabetes mellitus cases comprising of 400-750 mg/day of extract of root and 200-400 mg/day of extract of leaves of salacia oblonga (sapttarangi).

2. A composition for use in the prevention and management of endothelial dysfunction among type-II diabetes mellitus cases as claimed in Claim 1 wherein the composition may comprise of a known additive such as minerals, vitamin, salts, fillers (for capsulation or to prepare syrup) and binders.

3. A composition for use in the prevention and management of endothelial dysfunction among type-II diabetes mellitus cases as claimed in Claim 1 or 2 wherein the composition comprises of 625 mg/day of root and 325 mg/day of leaves of salacia oblonga (sapttarangi).

## Patentansprüche

1. Zusammensetzung zur Verwendung bei der Prävention und Behandlung von Endotheldysfunktion in Fällen von Diabetes mellitus Typ II, wobei die Zusammensetzung 400 - 750 mg/Tag Extrakt der Wurzel und 200 - 400 mg/Tag Extrakt der Blätter von *Salacia oblonga* (Saptarangi) umfasst.

2. Zusammensetzung zur Verwendung bei der Prävention und Behandlung von Endotheldysfunktion in Fällen von Diabetes mellitus Typ II nach Anspruch 1, wobei die Zusammensetzung einen bekannten Zusatzstoff umfassen kann, wie Mineralien, Vitamine, Salze, Füllstoffe (zur Verkapselung oder zum Herstellen eines Sirups) und Bindemittel.

3. Zusammensetzung zur Verwendung bei der Prävention und Behandlung von Endotheldysfunktion in Fällen von Diabetes mellitus Typ II nach Anspruch 1 oder 2, wobei die Zusammensetzung 625 mg/Tag der Wurzel und 325 mg/Tag der Blätter von *Salacia oblonga* (Saptarangi) umfasst.

## Revendications

1. Composition destinée à être utilisée dans la prévention et la prise en charge de la dysfonction endothéliale chez les patients atteints de diabète de type II, comprenant 400 à 750 mg/jour d'extrait de racine et 200 à 400 mg/jour d'extrait de feuilles de *Salacia oblonga* (saptarangi).

2. Composition destinée à être utilisée dans la prévention et la prise en charge de la dysfonction endothéliale chez les patients atteints de diabète de type II selon la revendication 1, dans laquelle la composition peut comprendre des additifs connus tels que des minéraux, des vitamines, des sels, des charges (pour la capsulation ou pour préparer des sirops) et des liants.

3. Composition destinée à être utilisée dans la prévention et la prise en charge de la dysfonction endothéliale chez les patients atteints de diabète de type II selon la revendication 1 ou la revendication 2, dans laquelle la composition comprend 625 mg/jour de racine et 325 mg/jour de feuilles de *Salacia oblonga* (saptarangi).
